# EUROPÄISCHE PATENTSCHRIFT

(11) **EP 1 915 358 B1**
(45) Veröffentlichungstag und Bekanntmachung des Hinweises auf die Patenterteilung: **17.12.2008**
(21) Anmeldenummer: 06778089.0
(22) Anmeldetag: 01.08.2006
(51) Int. Cl.: C07D 323/06

(54) **INTEGRIERTES VERFAHREN ZUR HERSTELLUNG VON TRIOXAN AUS FORMALDEHYD**
INTEGRATED METHOD FOR THE PRODUCTION OF TRIOXANE FROM FORMALDEHYDE
PROCEDE INTEGRE POUR PREPARER DU TRIOXANE A PARTIR DE FORMALDEHYDE

(30) Priorität: 08.08.2005 DE 102005037294
(43) Veröffentlichungstag der Anmeldung: 30.04.2008
(73) Patentinhaber: BASF SE, 67056 Ludwigshafen (DE)
(72) Erfinder: LANG, Neven, 68163 Mannheim (DE); STRÖFER, Eckhard, 68163 Mannheim (DE); STAMMER, Achim, 67251 Freinsheim (DE); FRIESE, Thorsten, 68163 Mannheim (DE); SIEGERT, Markus, 69126 Heidelberg (DE); HASSE, Hans, 67661 Kaiserslautern (DE); GRÜTZNER, Thomas, 70567 Stuttgart (DE); BLAGOV, Sergej, 70195 Stuttgart (DE)
(74) Vertreter: Isenbruck, Günter
(86) Internationale Anmeldenummer: PCT/EP2006/064876
(87) Internationale Veröffentlichungsnummer: WO 2007/017410

(56) Entgegenhaltungen:
- WO-A-20/05063353
- WO-A-20/05063733

## Beschreibung

Die Erfindung betrifft ein integriertes Verfahren zur Herstellung von Trioxan aus Formaldehyd.

Trioxan wird in der Regel durch Reaktivdestillation von wässriger Formaldehydlösung in Gegenwart saurer Katalysatoren hergestellt. Dabei fällt ein Trioxan, Formaldehyd und Wasser enthaltendes Gemisch als Destillat an. Aus diesem Gemisch wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln, abgetrennt.

DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem organischen, mit Wasser praktisch nicht mischbaren Extraktionsmittel für Trioxan beschickt, am anderen Ende mit Wasser. Zwischen den beiden Teilstrecken wird das zu trennende Destillat aus der Trioxan-Synthese zuführt. Auf der Seite der Lösungsmittelzuführung wird dabei eine wässrige Formaldehydlösung und auf der Seite der Wasserzuführung eine praktisch formaldehydfreie Lösung von Trioxan in dem Lösungsmittel erhalten.

Nachteilig an dieser Verfahrensweise ist der Anfall von Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in reines Trioxan einerseits und ein azeotropes Gemisch aus Trioxan, Wasser und Formaldehyd andererseits auftrennt. In einem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Diese Verfahrensweise ist sehr aufwendig. Insbesondere erfordert die Pervaporationseinheit hohe Investitionen.

WO-A-2005/063733 beschreibt ein Verfahren zur destillativen Abtrennung von Trioxan aus einem Trioxan/Formaldehyd/Wasser-Gemisch, bei dem ein Einsatzstrom mit einem Rückführstrom, welcher Trioxan, Formaldehyd und Wasser enthält, vermischt wird.

Aufgabe der Erfindung ist es, ein alternatives Verfahren zur Herstellung von Trioxan aus wässriger Formaldehydlösung unter Gewinnung von reinem Trioxan bereitzustellen. Aufgabe ist es insbesondere, ein Verfahren bereitzustellen, welches die Durchführung von Extraktionsschritten oder Pervaporationsschritten zur Gewinnung von reinem Trioxan vermeidet.

Diese Aufgabe wird durch ein integriertes Verfahren zur Herstellung von Trioxan aus Formaldehyd gelöst, welches folgende Schritte umfasst:
a) ein Wasser und Formaldehyd enthaltender Strom A1 und ein im Wesentlichen aus Wasser und Formaldehyd bestehender Rückführstrom B2 werden einem Trioxan-Synthesereaktor zugeführt, in welchem Formaldehyd zu Trioxan umgesetzt wird, wobei ein Trioxan, Wasser und Formaldehyd enthaltender Produktstrom A2 erhalten wird;
b) der Strom A2 und ein Trioxan, Wasser und Formaldehyd enthaltender Rückführstrom D1 werden einer ersten Destillationskolonne zugeführt und bei einem Druck im Bereich von 0,1 bis 2,5 bar destilliert, wobei ein an Trioxan angereicherter Strom B1 und der im Wesentlichen aus Wasser und Formaldehyd bestehende Strom B2 erhalten werden;
c) der Strom B1 wird einer zweiten Destillationskolonne zugeführt und bei einem Druck im Bereich von 0,2 bis 17,5 bar destilliert, wobei ein im Wesentlichen aus Trioxan bestehender Produktstrom C2 und ein Trioxan, Wasser und Formaldehyd enthaltender Strom C1 erhalten werden;
d) der Strom C1 wird einer dritten Destillationskolonne zugeführt und bei einem Druck im Bereich von 1 bis 10 bar destilliert, wobei der Trioxan, Wasser und Formaldehyd enthaltende Rückführstrom D1 und ein im Wesentlichen aus Wasser bestehender Strom D2 erhalten werden.

Im Wesentlichen aus einer oder mehreren Komponenten bestehend bedeutet dabei, dass diese Komponenten zu mindestens 90 Gew.%, bevorzugt zu mindestens 95 Gew.-% im entsprechenden Strom enthalten sind.

Es ist bekannt, dass Trioxan, Formaldehyd und Wasser ein ternäres Azeotrop bilden, welches bei einem Druck von 1 bar die Zusammensetzung 69 Gew.-% Trioxan, 5 Gew.-% Formaldehyd und 26 Gew.-% Wasser aufweist. Erfindungsgemäß wird das ternäre Azeotrop durch eine Druckwechseldestillation getrennt, indem eine erste und eine zweite Destillationsstufe bei verschiedenen Drücken durchgeführt werden. In einer ersten Destillationsstufe, welche bei niedrigerem Druck betrieben wird, wird das Ausgangsgemisch in ein Trioxan-reiches Trioxan/Wasser/Formaldehyd-Gemisch mit geringem Formaldehyd-Gehalt einerseits und ein im Wesentlichen Trioxan-freies Formaldehyd/Wasser-Gemisch andererseits aufgetrennt. Das Trioxan-reiche Trioxan/Wasser/Formalehyd-Gemisch wird anschließend in einer zweiten Destillationsstufe, welche bei hohem Druck durchgeführt wird, in ein Trioxan-reiches Trioxan/Wasser/Formaldehyd-Gemisch einerseits und im Wesentlichen reines Trioxan andererseits aufgetrennt. Erfindungsgemäß wird das Trioxan-reiche Trioxan/Wasser/Formaldehyd-Gemisch einer dritten Destillationsstufe zugeführt, die bei einem Druck betrieben wird, der zwischen dem Druck der ersten Destillationsstufe und der zweiten Destillationsstufe liegt. In der dritten Destillationsstufe werden ein Trioxan, Wasser und Formaldehyd enthaltender Strom und ein im Wesentlichen aus Wasser bestehender Strom erhalten. Der Trioxan, Wasser und Formaldehyd enthaltende Strom wird in die erste Destillationsstufe zurückgeführt. Hierdurch wird erreicht, dass im Wesentlichen alles bei der Synthese hergestellte Trioxan als Wertprodukt gewonnen wird.

Erfindungsgemäß umfasst jede Destillationsstufe eine Destillationskolonne. Als Destillationskolonne sind beliebige Destillationskolonnen, wie Packungs- oder Bodenkolonnen geeignet. Die Destillationskolonnen können beliebige Einbauten, Packungen oder Füllkörperschüttungen enthalten. Im Folgenden beziehen sich alle Druckangaben auf den Druck am Kopf der betreffenden Kolonne.

In einem ersten Verfahrensschritt a) wird ein Wasser und Formaldehyd enthaltender Strom A1 und ein im Wesentlichen aus Wasser und Formaldehyd bestehender Rückführstrom B2 einem Trioxan-Synthesereaktor zugeführt und reagieren gelassen, wobei ein Trioxan, Wasser und Formaldehyd enthaltender Produktstrom A2 gewonnen wird. Die Reaktion erfolgt dabei unter sauren Bedingungen.

Die Zufuhr der Ströme A1 und B2 kann dabei getrennt erfolgen. Es ist jedoch auch möglich, die Ströme A1 und B2 vor der Zufuhr in den Trioxan-Synthesereaktor zu mischen.

Im Allgemeinen enthält der Strom A1 50 bis 85 Gew.-% Formaldehyd und 15 bis 50 Gew.-% Wasser.

Das Verhältnis der Ströme A1 und B2 wird vorzugsweise so gewählt, dass dem Trioxan-Synthesereaktor insgesamt von 15 bis 70 Gew.-% Wasser und von 30 bis 85 Gew.-% Formaldehyd, besonders bevorzugt von 20 bis 63 Gew.-% Wasser und von 37 bis 80 Gew.-% Formaldehyd zugeführt werden.

Der Produktstrom A2 enthält im Allgemeinen 35 bis 84 Gew.-% Formaldehyd, 15 bis 45 Gew.-% Wasser und 1 bis 30 Gew.-% Trioxan.

In einer Ausführungsform des erfindungsgemäßen Verfahrens wird das Wasser/Formaldehydgemisch der Trioxan-Synthesestufe a) in Gegenwart saurer homogen oder heterogen vorliegender Katalysatoren wie Inenaustauscherharze, Zeolithe, Schwefelsäure und p-Toluolsulfonsäure bei einer Temperatur von im Allgemeinen 70 bis 130°C umgesetzt. Dabei kann in einer Reaktivdestillationskolonne oder einem Reaktivverdampfer gearbeitet werden. Das Produktgemisch aus Trioxan, Formaldehyd und Wasser fällt dann als dampfförmiger Brüdenabzugsstrom des Reaktiwerdampfers bzw. als Kopfabzugsstrom der Reaktivdestillationskolonne an. Die Trioxan-Synthese kann aber auch in einem Festbett- oder Fließbettreaktor an einem heterogenen Katalysator, z. B. einem Ionenaustauscherharz oder Zeolith, durchgeführt werden.

In einem sich an den Schritt a) anschließenden Schritt b) werden der Strom A2 und ein Trioxan, Wasser und Formaldehyd enthaltender Rückführstrom D1 einer ersten Destillationskolonne zugeführt und bei einem Druck von 0,1 bis 2,5 bar, vorzugsweise 0,3 bis 2 bar, besonders bevorzugt 0,4 bis 1,5 beispielsweise 1 bar destilliert, wobei ein mit Trioxan angereicherter Strom B1 und der im Wesentlichen aus Wasser und Formaldehyd bestehende Strom B2 erhalten werden.

Die erste Destillationskolonne enthält vorzugsweise 2 bis 50, besonders bevorzugt 4 bis 40 theoretische Stufen. Im Allgemeinen umfasst der Abtriebsteil der ersten Destillationskolonne mindestens 25% der Zahl der theoretischen Böden der Destillationskolonne. Vorzugsweise umfasst der Verstärkungsteil 50 bis 90% der theoretischen Stufen dieser Destillationskolonne.

Der an Trioxan angereicherte Strom B1 enthält im Allgemeinen 30 bis 80 Gew.-% Trioxan, 20 bis 69 Gew.-% Wasser und 1 bis 20 Gew.-% Formaldehyd. Bevorzugt enthält der an Trioxan angereicherte Strom B1 60 bis 75 Gew.-% Trioxan, 5 bis 35 Gew.-% Wasser und 5 bis 20 Gew.-% Formaldehyd. Der Strom B2 enthält im Allgemeinen 51 bis 85 Gew.-% Formaldehyd, 15 bis 49 Gew.-% Wasser und 0 bis 1 Gew.-% Trioxan. Vorzugsweise enthält der Strom B2 weniger als 0,5 Gew.-% Trioxan, besonders bevorzugt weniger als 0,1 Gew.-% Trioxan. Der Strom D1 enthält im Allgemeinen 5 bis 30 Gew.-% Formaldehyd, 5 bis 30 Gew.-% Wasser und 50 bis 80 Gew.-% Trioxan. Vorzugsweise enthält der Strom D1 5 bis 20 Gew.-% Formaldehyd, 10 bis 40 Gew.-% Wasser und 55 bis 70 Gew.-% Trioxan.

Die Ströme A2 und D1 werden der ersten Destillationskolonne vorzugsweise als Seitenzuläufe zugeführt. Der Strom B1 wird der ersten Destillationskolonne vorzugsweise als Kopfabzugsstrom und der Strom B2 als Sumpfabzugsstrom entnommen.

In einer, bevorzugten Ausführungsform wird der Strom A2 der ersten Destillationskolonne als Seitenzulauf im Abtriebsteil oder im Sumpf der Kolonne zugeführt und der Strom D1 als Seitenzulauf im Verstärkungsteil. In einer weiteren Ausführungsform können der Strom A2 und der Strom D1 auch vor Zugabe in die erste Destillationskolonne gemischt werden. Die Zugabe erfolgt dann vorzugsweise als gemeinsamer Seitenzulauf.

In einer weiteren Ausführungsform des erfindungsgemäßen Verfahrens werden die Trioxan-Synthesestufe a) und die erste Destillationsstufe b) gemeinsam als Reaktivdestillation in einer Reaktionskolonne durchgeführt. Diese kann im Abtriebsteil ein Katalysator-Festbett aus einem heterogenen Katalysator enthalten. Alternativ kann die Reaktivdestillation auch in Gegenwart eines homogenen Katalysators durchgeführt werden, wobei ein saurer Katalysator zusammen mit der wässrigen Formaldehyd-Lösung im Kolonnensumpf vorliegt. Bevorzugt wird die Reaktivdestillation in Gegenwart eines homogenen sauren Katalysators, der zusammen mit der wässrigen Formaldehyd-Lösung im Kolonnensumpf vorliegt, durchgeführt.

In einem sich an den Schritt b) anschließenden Verfahrensschritt c) wird der an Trioxan angereicherte Strom B1 einer zweiten Destillationskolonne zugeführt und bei einem Druck von 0,2 bis 17,5 bar destilliert, wobei ein im Wesentlichen aus reinem Trioxan bestehender Produktstrom C2 und ein Trioxan, Wasser und Formaldehyd enthaltender Strom C1 erhalten werden.

Die zweite Destillationskolonne umfasst im Allgemeinen mindestens 2 theoretische Stufen, vorzugsweise 10 bis 50 theoretischen Stufen. Im Allgemeinen umfasst der Abtriebsteil dieser Destillationskolonne 25 bis 90%, vorzugsweise 50 bis 75% der theoretischen Stufen dieser Kolonne.

Der Druck in der zweiten Destillationskolonne ist um mindestens 0,1 bar höher als in der ersten Destillationskolonne. Im Allgemeinen beträgt diese Druckdifferenz 0,5 bis 10 bar, vorzugsweise 1 bis 7 bar. Die zweite Destillationskolonne wird vorzugsweise bei einem Druck zwischen 2 und 10 bar, besonders bevorzugt bei einem Druck zwischen 2 und 7 bar betrieben.

Der Produktstrom C2 enthält im Allgemeinen 95 bis 100 Gew.-%, vorzugsweise 99 bis 100 Gew.-% Trioxan und 0 bis 5 Gew.%, vorzugsweise 0 bis 1 Gew.-% Wasser. Besonders bevorzugt ist der Gehalt an Wasser im Produktstrom <0,1%. Er kann sogar <0,01% sein. Der Strom C1 enthält beispielsweise 5 bis 20 Gew.-% Formaldehyd, 15 bis 35 Gew.-% Wasser und 50 bis 75 Gew.-% Trioxan.

Vorzugsweise wird der zweiten Destillationskolonne der Strom B1 als Seitenzulauf, bevorzugt als Seitenzulauf im Abtriebsteil der zweiten Destillationskolonne zugeführt. Der Strom C1 wird der zweiten Destillationskolonne vorzugsweise als Kopfabzugsstrom und der Produktstrom C2 als Sumpfabzugsstrom entnommen.

In dem sich an den Schritt c) anschließenden Schritt d) wird der Strom C1 einer dritten Destillationskolonne zugeführt und bei einem Druck im Bereich von 1 bis 10 bar destilliert, wobei der Trioxan, Wasser und Formaldehyd enthaltende Rückführstrom D1 und ein im Wesentlichen aus Wasser bestehender Strom D2 erhalten werden.

Die dritte Destillationsstufe wird vorzugsweise bei einem Druck zwischen 1 und 5 bar durchgeführt.

Die dritte Destillationskolonne weist mindestens zwei theoretische Stufen, vorzugsweise 10 bis 50 theoretische Stufen auf. Im Allgemeinen umfasst der Abtriebsteil dieser Destillationskolonne 25 bis 90%, vorzugsweise 50 bis 75% der theoretischen Stufen dieser Kolonne.

Vorzugsweise wird der Strom C1 der dritten Destillationskolonne als Seitenzulauf im Abtriebsteil der Kolonne zugeführt. Der Rückführstrom D1 fällt im Allgemeinen am Kopf der dritten Destillationskolonne und der im Wesentlichen aus Wasser bestehende Strom D2 als Sumpfabzugsstrom oder als Seitenabzugsstrom im Abtriebsteil der Kolonne an.

In einer bevorzugten Ausführungsform wird bei dem Verfahren zur Herstellung von Trioxan aus Formaldehyd in einem weiteren Schritt ein Wasser und Formaldehyd enthaltender Einspeisungsstrom E1 einer Formaldehyd-Aufkonzentrierungseinheit zugeführt. Der Aufkonzentrierungseinheit werden der Strom A1 als Formaldehyd-reicher Sumpfstrom und ein Formaldehyd-armer Strom E2 als Kopf- oder Brüdenabzugsstrom entnommen.

Die Aufkonzentrierung des Wasser und Formaldehyd enthaltenden Einspeisungsstromes E1 erfolgt im Allgemeinen in einer Destillationskolonne oder in einem Verdampfer. Bevorzugt wird die Aufkonzentrierung in einem Verdampfer, besonders bevorzugt in einem kontinuierlichen Verdampfer durchgeführt. Geeignete kontinuierliche Verdampfer sind z. B. Umlaufverdampfer, Fallfilmverdampfer, Wendelrohrverdampfer oder Dünnschichtverdampfer. Besonders bevorzugt werden Fallfilmverdampfer zur Aufkonzentrierung des Wasser/Formaldehyd-Gemisches eingesetzt. Der Fallfilmverdampfer wird dabei im Allgemeinen bei einem Druck von 50 bis 200 mbar und einer Temperatur von 40 bis 75°C betrieben.

Der Aufkonzentrierungsschritt kann beispielsweise wie in DE-A 199 25 870 beschrieben durchgeführt werden.

Der bei der Aufkonzentrierung erhaltene Formaldehyd-reiche Strom A1 wird im Allgemeinen als Sumpfabzugsstrom entnommen, der Formaldehyd-arme Strom E2 wird als Kopf- oder Brüdenabzugsstrom entnommen.

Bei Verwendung einer Destillationskolonne zur Aufkonzentrierung wird der Wasser und Formaldehyd enthaltende Einspeisungsstrom E1 vorzugsweise als Seitenzulauf zugeführt.

In einer bevorzugten Ausführungsform wird der Formaldehyd-arme Strom E2 der dritten Destillationskolonne zugeführt. Bevorzugt wird der Strom E2 der dritten Destillationskolonne als Seitenzulauf am Kolonnenkopf zugeführt. In einer weiteren Ausführungsform können der Strom E2 und der Strom C1 zunächst gemischt werden und anschließend als gemeinsamer Seitenzulauf der dritten Destillationskolonne zugegeben werden.

Neben Wasser, Formaldehyd und Trioxan können insbesondere in den Strömen A2, B1, C1 und D2 noch bis zu 15 Gew.%, im Allgemeinen 1 bis 10 Gew.-% Leichtsieder enthalten sein. Übliche Leichtsieder, die bei der Trioxan-Synthese und der nachfolgenden destillativen Trennung gebildet werden können, sind Methylformiat, Methylal, Dimethoxydimethylether, Trimethoxydimethylether, Methanol, Ameisensäure sowie weitere Halb- und Vollacetale. Zur Abtrennung dieser Leichtsieder kann optional nach der ersten Destillationsstufe b) eine weitere Destillationsstufe (Leichtsieder-Abtrennstufe) durchgeführt werden. Dabei werden die Leichtsieder vorzugsweise über den Kopf der Leichtsieder-Abtrennkolonne, welche vorzugsweise bei einem Druck von 1 bis 3 bar betrieben wird, abgetrennt. Im Allgemeinen weist die Leichtsieder-Abtrennkolonne mindestens 5 theoretische Stufen, vorzugsweise 15 bis 50 theoretische Stufen auf. Vorzugsweise umfasst der Abtriebsteil dieser Kolonne 25 bis 90% der theoretischen Stufen dieser Kolonne. Der Strom B1 wird dieser Leichtsieder-Abtrennkolonne als Seitenzulauf zugeführt und der von den Leichtsiedern befreite Strom B1' wird im Allgemeinen als Sumpfabzugsstrom enthalten. Wird die Leichtsieder-Abtrennung durchgeführt, so wird der Strom B1' als Strom B1 der nachfolgenden zweiten Destillationskolonne zugeführt.

Das erhaltene Rein-Trioxan, dessen Reinheit >99 Gew.%, bevorzugt >99,5 Gew.-% oder sogar >99,8 Gew.-% betragen kann, wird vorzugsweise zur Herstellung von Polyoxymethylen (POM), Polyoxymethylenderivaten wie Polyoxymethylendimethylether (POMDME) und Diaminodiphenylmethan (MDA) verwendet.

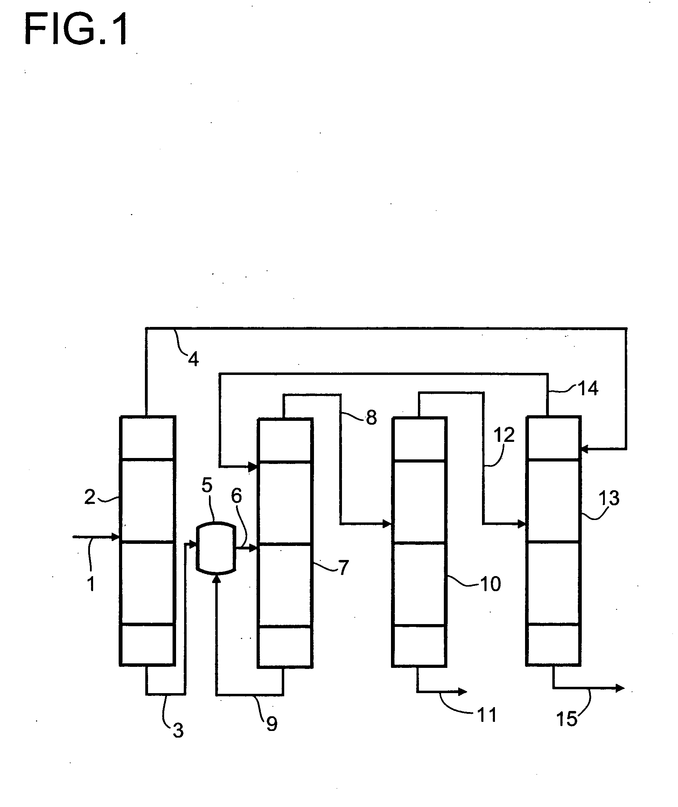

Im Folgenden wird die Erfindung anhand der Zeichnung näher beschrieben.

Die einzige Figur zeigt ein Verfahrensfließbild des erfindungsgemäßen Verfahrens.

Eine wässrige Formaldehyd-Lösung 1 (Strom E1) wird einer Aufkonzentrierungseinheit 2 zugegeben. Die Aufkonzentrierungseinheit 2 kann dabei eine beliebige Destillationskolonne, z. B. eine Bodenkolonne, Packungskolonne oder Füllkörperkolonne oder ein kontinuierlicher Verdampfer, z. B. ein Umlaufverdampfer, Fallfilmverdampfer, Wendelrohrverdampfer oder Dünnschichtverdampfer sein. Bevorzugt ist die Aufkonzentrierungseinheit 2 ein Fallfilmverdampfer. Aus der Aufkonzentrierungseinheit 2 werden ein Formaldehyd-reicher Sumpfabzugsstrom 3 (Strom A1) und ein Formaldehyd-armer wässriger Brüdenstrom als Kopfabzugsstrom 4 (Strom E2) erhalten. Der Formaldehyd-reiche Sumpfabzugsstrom 3 wird einem Trioxan-Synthesereaktor 5 zugeführt. Im Trioxan-Synthesereaktor 5 wird die wässrige Formaldehydlösung in Gegenwart eines sauren homogen oder heterogen vorliegenden Katalysators zu Trioxan umgesetzt.

Aus dem Trioxan-Synthesereaktor 5 wird ein Trioxan, Formaldehyd und Wasser enthaltender Strom 6 (Strom A2) als Seitenzulauf einer ersten Destillationskolonne 7 zugeführt. In der ersten Destillationskolonne 7 wird der Strom 6 in einen an Trioxan angereicherten Strom 8 (Strom B1), der der ersten Destillationskolonne 7 als Kopfabzugsstrom entnommen wird, und einen als Sumpfabzug anfallenden, im Wesentlichen aus Wasser und Formaldehyd bestehenden Strom 9 (Strom B2) aufgetrennt. Der am Sumpf anfallende Strom 9 (Strom B2) wird in den Trioxan-Synthesereaktor 5 zurückgeführt.

Der am Kopf der ersten Destillationskolonne 7 anfallende Strom 8 (Strom B1) wird einer zweiten Destillationskolonne 10 zugeführt. Dieser wird in der zweiten Destillationskolonne 10 in einen im Wesentlichen Trioxan enthaltenden Produktstrom 11 (Strom C2) sowie einen Trioxan, Wasser und Formaldehyd enthaltenden Strom 12 (Strom C1), der am Kopf der zweiten Destillationskolonne 10 abgezogen wird, aufgetrennt. Der Strom 14 wird über einen Seitenzulauf einer dritten Destillationskolonne 13 zugeführt. In der dritten Destillationskolonne 13 erfolgt eine Auftrennung des Stromes 12 in einen Trioxan, Formaldehyd und Wasser enthaltenden, am Kopf anfallenden Rückführstrom 14 (Strom D1) und einen im Wesentlichen aus Wasser bestehenden Strom 15 (Strom D2), der am Sumpf der dritten Destillationskolonne abgezogen wird.

Der Rückführstrom 14 (Strom D1) wird der ersten Destillationskolonne 7 zugeführt. Die Zufuhr des Rückführstromes 14 erfolgt dabei über einen Seitenzulauf in die erste Destillationskolonne 7.

Zusätzlich zu dem Strom 12 (Strom C1) wird der dritten Destillationskolonne 13 der am Kopf der Aufkonzentrierungseinheit 2 abgezogene Formaldehyd-arme Strom 4 (Strom E2) zugeführt. Die Zufuhr erfolgt dabei vorzugsweise als Seitenzulauf am Kolonnenkopf der dritten Destillatiönskolonne 13.

### Beispiel

Einer als Fallfilmverdampfer ausgebildeten Aufkonzentriereinheit 2 wird eine wässrige Formaldehyd-Lösung 1 aus 37 Gew.-% Formaldehyd und 63 Gew.-% Wasser zugeführt. Der Fallfilmverdampfer wird bei einem Druck von 100 mbar und einer Temperatur von 50°C betrieben. Dem Sumpf des Fallfilmverdampfers wird ein Sumpfabzugsstrom 3 mit 50 Gew.-% Formaldehyd und 50 Gew.-% Wasser entnommen. Der Kopfabzugsstrom 4 des Fallfilmverdampfers enthält 20 Gew.-% Formaldehyd, der Rest ist Wasser.

Der Sumpfabzugsstrom 3 wird dem Trioxan-Synthesereaktor 5 zugeführt. Dieser ist als Rührbehälter ausgeführt und wird bei einer Temperatur von 108°C betrieben. Der Austragsstrom 6 enthält 9 Gew.-% Trioxan und 66 Gew.-% Formaldehyd, der Rest ist Wasser.

Der Strom 6 wird der ersten Destillationskolonne 7 auf den fünften Boden zugeführt. Ebenso wird dieser Destillationskolonne 7 der Strom 14 als Rückführung aus dem Prozess auf den 20. Boden zurückgeführt. Die erste Destillationskolonne 7 wird bei einem Druck von 1 bar betrieben. Die Kopftemperatur beträgt ca. 101°C, die Sumpftemperatur beträgt ca. 104°C. Sie enthält 30 Böden. Der ersten Destillationskolonne wird am Sumpf ein Strom 9 mit einer Konzentration von 80 Gew.-% Formaldehyd und 20 Gew.-% Wasser entnommen. Am Kopf der Kolonne 7 wird der Strom 8 mit einer Konzentration von 66 Gew.-% Trioxan, 7 Gew.-% Formaldehyd und 27 Gew.-% Wasser entnommen.

Der Strom 8 wird der zweiten Destillationskolonne 10 zugeführt. Diese wird bei einem Druck von 4 bar betrieben. Die Kopftemperatur beträgt ca. 142°C, die Sumpftemperatur beträgt ca. 166°C. Die Kolonne hat 40 Böden, der Strom 8 wird dem Boden 20 zugeführt. Am Kopf der Kolonne 10 wird ein Strom 12 mit 64 Gew.-% Trioxan, 8 Gew.-% Formaldehyd und 28 Gew.-% Wasser entnommen. Am Sumpf wird der Produktstrom 11 mit mehr als 99 Gew.-% Trioxan abgenommen.

Der Kopfstrom 12 wird der dritten Destillationskolonne 13 auf den 24. Boden zugeführt. Der dritten Destillationskolonne 13 wird ebenfalls der Strom 4 aus dem Fallfilmverdampfer 2 auf den 36. Boden zugeführt. Die dritte Destillationskolonne 13 enthält 48 Böden und wird bei einem Druck von 2,5 bar betrieben. Die Kopftemperatur beträgt ca. 127°C, die Sumpftemperatur beträgt ca. 131°C. Am Sumpf dieser Kolonne wird der Strom 15 entnommen. Dieser Strom enthält mehr als 98 Gew.-% Wasser. Der Kopfstrom 14 enthält 66 Gew.-% Trioxan, 8 Gew.-% Formaldehyd und 26 Gew.-% Wasser. Dieser Strom 14 wird in die erste Destillationskolonne 7 zurückgeführt.

## Patentansprüche

1. Integriertes Verfahren zur Herstellung von Trioxan aus Formaldehyd, welches folgende Schritte umfasst:
a) ein Wasser und Formaldehyd enthaltender Strom A1 und ein im Wesentlichen aus Wasser und Formaldehyd bestehender Rückführstrom B2 werden einem Trioxan-Synthesereaktor zugeführt, in welchem das Formaldehyd zu Trioxan umgesetzt wird, wobei ein Trioxan, Wasser und Formaldehyd enthaltender Produktstrom A2 erhalten wird;
b) der Strom A2 *wird als Seitenzulauf in den Abtriebsteil* und ein Trioxan, Wasser und Formaldehyd enthaltender Rückführstrom D1 *wird als Seitenzulauf in den Verstärkungsteil* einer ersten Destillationskolonne zugeführt und bei einem Druck im Bereich von 0,1 bis 2,5 bar destilliert, wobei ein an Trioxan angereicherter Strom B1 und der im Wesentlichen aus Wasser und Formaldehyd bestehende Strom B2 erhalten werden;
c) der Strom B1 wird einer zweiten Destillationskolonne zugeführt und bei einem Druck im Bereich von 0,2 bis 17,5 bar destilliert, wobei ein im Wesentlichen aus Trioxan bestehender Produktstrom C2 und ein Trioxan, Wasser und Formaldehyd enthaltender Strom C1 erhalten werden;
d) der Strom C1 wird einer dritten Destillationskolonne zugeführt und bei einem Druck im Bereich von 1 bis 10 bar destilliert, wobei der Trioxan, Wasser und Formaldehyd enthaltende Rückführstrom D1 und ein im Wesentlichen aus Wasser bestehender Strom D2 erhalten werden.

2. Verfahren nach Anspruch 1, **dadurch gekennzeichnet, dass** die Destillation b) bei einem Druck im Bereich von 0,4 bis 1,5 bar, die Destillation c) bei einem Druck im Bereich von 2 bis 7 bar und die Destillation d) bei einem Druck im Bereich von 1 bis 5 bar durchgeführt werden.

3. Verfahren nach Anspruch 1 oder 2, **dadurch gekennzeichnet, dass** der ersten Destillationskolonne der Strom B1 als Kopfabzugsstrom und der Strom B2 als Sumpfabzugsstrom entnommen werden und der Strom A2 als Zulauf im Sumpf der Kolonne oder als Seitenzulauf und der Strom D1 als Seitenzulauf zugeführt werden.

4. Verfahren nach einem der Ansprüche 1 bis 3, **dadurch gekennzeichnet, dass** der zweiten Destillationskolonne der Strom B1 als Seitenzulauf zugeführt wird und der Strom C1 als Kopfabzugsstrom und der Strom C2 als Sumpfabzugsstrom entnommen werden.

5. Verfahren nach einem der Ansprüche 1 bis 4, **dadurch gekennzeichnet, dass** der dritten Destillationskolonne der Strom C1 als Seitenzulauf zugeführt wird und der Strom D1 als Kopfabzugstrom und der Strom D2 als Sumpfabzugsstrom entnommen werden.

6. Verfahren nach einem der Ansprüche 1 bis 5, **dadurch gekennzeichnet, dass** in einem weiteren Schritt ein Wasser und Formaldehyd enthaltender Einspeisungsstrom E1 einer Formaldehyd-Aufkonzentrierungseinheit zugeführt wird, der Strom A1 der Aufkonzentrierungseinheit als Formaldehyd-reicher Sumpfstrom und ein Formaldehyd-armer Strom E2 als Kopf- oder Brüdenabzugsstrom entnommen werden.

7. Verfahren nach Anspruch 6, **dadurch gekennzeichnet, dass** der Strom E2 der dritten Destillationskolonne zugeführt wird.

8. Verfahren nach Anspruch 6, **dadurch gekennzeichnet, dass** der Strom E2 der dritten Destillationskolonne als Seitenzulauf am Kolonnenkopf zugeführt wird.

9. Verfahren nach Anspruch 6, **dadurch gekennzeichnet, dass** der Strom E2 und der Strom C1 vor Zugabe in die dritte Destillationskolonne gemischt werden und dann als gemeinsamer Seitenzulauf der dritten Destillationskolonne zugeführt werden.

10. Verfahren nach Anspruch 6, **dadurch gekennzeichnet, dass** die Formaldehyd-Aufkonzentrierungseinheit eine Destillationskolonne ist.

11. Verfahren nach Anspruch 6, **dadurch gekennzeichnet, dass** die Formaldehyd-Aufkonzentrierungseinheit ein Verdampfer ist.

12. Verfahren nach Anspruch 11, **dadurch gekennzeichnet, dass** der Verdampfer ein Fallfilmverdampfer ist.

## Claims

1. An integrated process for preparing trioxane from formaldehyde, which comprises the following steps:
a) a stream A1 comprising water and formaldehyde and a recycle stream B2 consisting substantially of water and formaldehyde are fed to a trioxane synthesis reactor in which the formaldehyde is converted to trioxane to obtain a product stream A2 comprising trioxane, water and formaldehyde;
b) stream A2 is fed as a side feed in the stripping section and a recycle stream D1 comprising trioxane, water and formaldehyde is fed as a side feed in the rectifying section to a first distillation column and distilled at a pressure in the range from 0.1 to 2.5 bar to obtain a stream B1 enriched in trioxane, and the stream B2 consisting substantially of water and formaldehyde;
c) stream B1 is fed to a second distillation column and distilled at a pressure in the range from 0.2 to 17.5 bar to obtain a product stream C2 consisting substantially of trioxane, and a stream C1 comprising trioxane, water and formaldehyde;
d) stream C1 is fed to a third distillation column and distilled at a pressure in the range from 1 to 10 bar to obtain the recycle stream D1 comprising trioxane, water and formaldehyde, and a stream D2 consisting substantially of water.

2. The process according to claim 1, wherein the distillation b) is carried out at a pressure in the range from 0.4 to 1.5 bar, the distillation c) at a pressure in the range from 2 to 7 bar and the distillation d) at a pressure in the range from 1 to 5 bar.

3. The process according to claim 1 or 2, wherein stream B1 is withdrawn as a top draw stream and stream B2 as a bottom draw stream from the first distillation column and stream A2 is fed as a feed in the bottom of the column or as a side feed and stream D1 as a side feed.

4. The process according to any of claims 1 to 3, wherein stream B1 is fed as a side feed to the second distillation column and stream C1 is withdrawn as a top draw stream and stream C2 as a bottom draw stream.

5. The process according to any of claims 1 to 4, wherein stream C1 is fed as a side feed to the third distillation column, and stream D1 is withdrawn as a top draw stream and stream D2 as a bottom draw stream.

6. The process according to any of claims 1 to 5, wherein, in a further step, a feed stream E1 comprising water and formaldehyde is fed to a formaldehyde concentration unit, stream A1 is withdrawn from the concentration unit as a formaldehyde-rich bottom stream and a low-formaldehyde stream E2 is withdrawn as a top or vapor draw stream.

7. The process according to claim 6, wherein stream E2 is fed to the third distillation stage.

8. The process according to claim 6, wherein stream E2 is fed to the third distillation column as a side feed at the top of the column.

9. The process according to claim 6, wherein stream E2 and stream C1 are mixed before addition to the third distillation column and then fed as a combined side feed to the third distillation column.

10. The process according to claim 6, wherein the formaldehyde concentration unit is a distillation column.

11. The process according to claim 6, wherein the formaldehyde concentration unit is an evaporator.

12. The process according to claim 11, wherein the evaporator is a falling-film evaporator.

## Revendications

1. Procédé intégré de fabrication de trioxane à partir de formaldéhyde, qui comprend les étapes suivantes :
a) un courant A1 contenant de l'eau et du formaldéhyde et un courant de recyclage B2 constitué essentiellement d'eau et de formaldéhyde sont introduits dans un réacteur de synthèse de trioxane, dans lequel le formaldéhyde est transformé en trioxane, un courant de produit A2 contenant du trioxane, de l'eau et du formaldéhyde étant obtenu ;
b) le courant A2 est introduit en tant qu'alimentation latérale dans la zone de rectification d'une première colonne de distillation et un courant de recyclage D1 contenant du trioxane, de l'eau et du formaldéhyde est introduit en tant qu'alimentation latérale dans la partie d'enrichissement et ils sont distillés à une pression de 0,1 à 2,5 bars, un courant B1 enrichi en trioxane et un courant B2 constitué essentiellement d'eau et de formaldéhyde étant obtenus ;
c) le courant B1 est introduit dans une deuxième colonne de distillation et distillé à une pression de 0,2 à 17,5 bars, un courant de produit C2 constitué essentiellement de trioxane et un courant C1 contenant du trioxane, de l'eau et du formaldéhyde étant obtenus ;
d) le courant C1 est introduit dans une troisième colonne de distillation et distillé à une pression de 1 à 10-bars, un courant de recyclage D1 contenant du trioxane, de l'eau et du formaldéhyde et un courant D2 contenant essentiellement de l'eau étant obtenus.

2. Procédé selon la revendication 1, **caractérisé en ce que** la distillation b) est réalisée à une pression de 0,4 à 1,5 bar, la distillation c) à une pression de 2 à 7 bars et la distillation d) à une pression de 1 à 5 bars.

3. Procédé selon la revendication 1 ou 2, **caractérisé en ce que** dans la première colonne de distillation, le courant B1 est déchargé en tant que courant de sortie de tête et le courant B2 en tant que courant de sortie de fond et le courant A2 est introduit en tant qu'alimentation dans le fond de la colonne ou en tant qu'alimentation latérale et le courant D1 en tant qu'alimentation latérale.

4. Procédé selon l'une quelconque des revendications 1 à 3, **caractérisé en ce que** dans la deuxième colonne de distillation, le courant B1 est introduit en tant qu'alimentation latérale et le courant C1 est déchargé en tant que courant de sortie de tête et le courant C2 en tant que courant de sortie de fond.

5. Procédé selon l'une quelconque des revendications 1 à 4, **caractérisé en ce que** dans la troisième colonne de distillation, le courant C1 est introduit en tant que courant latéral et le courant D1 est déchargé en tant que courant de sortie de tête et le courant D2 en tant que courant de sortie de fond.

6. Procédé selon l'une quelconque des revendications 1 à 5, **caractérisé en ce que** lors d'une étape supplémentaire, un courant d'alimentation E1 contenant de l'eau et du formaldéhyde est introduit dans une unité de concentration du formaldéhyde, le courant A1 étant déchargé de l'unité de concentration en tant que courant de fond riche en formaldéhyde et un courant E2 pauvre en formaldéhyde en tant que courant de sortie de tête ou de buées.

7. Procédé selon la revendication 6, **caractérisé en ce que** le courant E2 est introduit dans la troisième colonne de distillation.

8. Procédé selon la revendication 6, **caractérisé en ce que** le courant E2 est introduit dans la troisième colonne de distillation en tant qu'alimentation latérale au niveau de la tête de la colonne.

9. Procédé selon la revendication 6, **caractérisé en ce que** le courant E2 et le courant C1 sont mélangés avant l'introduction dans la troisième colonne de distillation, puis sont introduits ensemble en tant qu'alimentation latérale de la troisième colonne de distillation.

10. Procédé selon la revendication 6, **caractérisé en ce que** l'unité de concentration du formaldéhyde est une colonne de distillation.

11. Procédé selon la revendication 6, **caractérisé en ce que** l'unité de concentration du formaldéhyde est un évaporateur.

12. Procédé selon la revendication 11, **caractérisé en ce que** l'évaporateur est un évaporateur à film tombant.
